# EUROPEAN PATENT APPLICATION

(11) **EP 0 614 983 A2**
(43) Date of publication of application: **14.09.1994**
(21) Application number: 94301462.1
(22) Date of filing: 01.03.1994
(51) Int. Cl.: C12P 7/62, C07C 59/08, C12P 7/56

(54) **Method for the production of lactic acid and lactic esters**

(30) Priority: 02.03.1993 JP 41334/93; 16.09.1993 JP 230428/93
(71) Applicant: MUSASHINO CHEMICAL LABORATORY Ltd., Tokyo (JP)
(72) Inventor: Kumagai, Akira, Hidaka-shi, Saitama-ken (JP); Yaguchi, Masaaki, Tokyo (JP); Arimura, Tomohiro, Sagamihara-shi, Kanagawa-ken (JP); Miura, Shigenobu, Tokyo (JP)
(74) Representative: Rees, David Christopher

(57) **Abstract**

A method for producing a lactic ester inexpensively by microorganic fermentation of lactic acid with a simple apparatus is provided, which method effects adjustment of the pH of a culture medium during the course of the fermentation with ammonia and, after the end of the fermentation, recovers the ammonia for reuse to lower the cost required for the fermentation and decrease the amount of a by-product of fermentation.

In a process involving fermentation of lactic acid with a microorganism, a method for the production of a lactic ester which comprises carrying out the adjustment of pH of a culture medium undergoing the fermentation of lactic acid with ammonia, adding an alcohol having four or five carbon atoms to at least one solution selected from the group consisting of a solution of ammonium lactate obtained by the fermentation, the concentrate of the solution of ammonium lactate mentioned above, and the solution resulting from separation of solid substances including the microorganic cells used for the fermentation from either of the solutions and heating the resultant mixture thereby inducing dehydration and esterification of lactic acid with the alcohol and, at the same time, effecting liberation and recovery of ammonia, and adding a mineral acid to the solution obtained by the esterification reaction and heating and dehydrating the resultant mixture in an acidic state thereby promoting and completing the esterification of the lactic acid with the alcohol.

## Description

This invention relates to a novel method for the production of lactic acid and lactic esters.

### Description of the Prior Art

At present, lactic acid is used as a food additive in the production of Japanese sake, refreshing beverages, pickled vegetables, soy sauce, bread, and beer. In terms of industrial consumption, it is used for the production of leather, textiles, plastics, medicines, and agricultural pesticides.

Recently, derivatives of lactic acid or intermediates for synthesis which are lactic esters such as ethyl lactate and butyl lactate have been finding extensive utility for solvents and detergents of the kind featuring high safety. Polylactic acid which is a polymer of lactic acid is expected to find growing utility as a biodegradable polymer.

The lactic acid available today is known in two types; the lactic acid obtained from a petrochemical product by chemical synthesis and the lactic acid obtained by fermentation. The lactic acid which is obtained from a petrochemical product by chemical synthesis is generally purified in the form of a lactic ester. The lactic ester, therefore, has high purity. It is, however, racemic in form and devoid of optical activity. The lactic acid which is produced by fermentation occurs in an L- or D-optically active form and racemic form. A desired optically active form of this lactic acid can be prepared freely, depending on the kind of a microorganism to be used for fermentation. Generally, such lactates are not easy to purify, inferior in quality to their synthetically produced countertypes, and unfit for the production of medicines or agricultural pesticides. To be specific, for use as food additives and for general industrial consumption, such racemic forms of lactic acid or lactic esters as are produced by the chemical synthesis can be used quite safely. As raw materials for medicines and agricultural pesticides or as a raw material for the polylactic acid accepted as a biodegradable polymer, however, the lactic acid or the lactic ester is required to possess high optical activity and high purity.

The lactic acid which possesses such high optical activity as mentioned above, however, cannot be produced except by a process of fermentation in the existing circumstances. Today, the lactic acid manufactured by fermentation and possessed of optical activity is obtained by a method of fermentation using glucide or starch as a raw material and causing lactic acid bacteria to assimilate the raw material. This method comprises preparing a culture medium incorporating therein whey, corn steep liquor, or yeast extract as a nutrient for the lactic acid bacteria and calcium carbonate as a neutralizing agent for the lactic acid to be formed, inoculating lactic acid bacteria to the culture medium and inducing fermentation productive of lactic acid, adding sulfuric acid to the fermentation solution containing calcium lactate at the end of the fermentation thereby inducing precipitation of gypsum and liberation of lactic acid, then filtering the resultant fermentation solution thereby removing such solid substances as gypsum and bacterial cells and obtaining lactic acid in a crude form, and purifying the crude lactic acid through the steps of ether extraction and treatment with activated carbon. This method is still incapable of easily manufacturing lactic acid or lactic esters which possess high purity and high optical activity required of a raw material for medicines and agricultural pesticides or of a raw material for polylactic acid usable as a biodegradable polymer material.

Thus, the desirability of developing a method capable of easily producing lactic acid possessing high purity and high optical activity has been finding enthusiastic recognition in the field of medicines and the field of agriculture.

As measures for easy production of lactic acid possessing high purity and high optical activity, a method which comprises carrying out the fermentation while using ammonia or sodium hydroxide in the place of calcium carbonate as the neutralizing agent for the lactic acid to be formed thereby inducing formation of ammonium lactate or sodium lactate, ultrafiltering the fermentation solution thereby separating the lactate just mentioned from the other culture medium components, and then obtaining free lactic acid by ion exchange (JP-A-63-38) and a method which comprises converting lactic acid obtained in a crude form into such a lactate as zinc lactate which has a relatively low solubility and purifying the lactate to a high degree by means of crystallization and consequently obtaining free lactic acid (JP-A-63-188632) have been disclosed.

The methods described above, however, entail such problems as shown in (1) to (3) below.

(1) The adjustment of pH of the culture medium in the process of fermentation requires virtually the equivalent amount of an alkali as that of the lactic acid to be formed. The methods mentioned above, for the purpose of liberating lactic acid from the formed lactate, call for an equivalent amount of an acid. They must further dispose appropriately of by-produced gypsum and inorganic salts such as ammonium sulfate and sodium sulfate. They do not permit easy production of fully purified lactic acid.

(2) The methods resorting to separation with a membrane or ion exchange as described above are still incapable of producing lactic acid purified to a high degree. For the purpose of obtaining lactic acid which exhibits high purity, avoids emitting offensive odor, and enjoys ample thermal stability, these methods necessitate numerous additional treatments such as, for example, a procedure which comprises esterifying lactic acid obtained in a crude form with such an alcohol as ethanol or methanol, then distilling the resultant lactic ester thereby forming purified lactic ester, and hydrolyzing the purified lactic ester.

The esterification and the distillation which are required for the production of lactic acid of high purity must be preceded by a preliminary treatment for rough purification. Thus, these methods do not prove economical. Further, as pointed out in JP-A-58-56,690, the impurities originating in a glucide, bacterial cells, and a nutrient source and entrained in the lactic acid solution resulting from the rough purification are suffered to deposit copiously on the walls of a distillation column and those of a reaction kettle as observed when the lactic acid solution obtained by the ordinary method of fermentation and containing impurities is subjected to esterification. The fast deposition of these impurities on the walls of the reaction kettle entails an operational trouble and renders it difficult to obtain lactic acid of high purity.

(3) The method which effects the purification of crude lactic acid by crystallization and separation of the lactic acid in the form of calcium lactate or zinc lactate deserves no rating as economical because the produced lactic acid is lost so heavily in the mother liquor as to degrade the yield of lactic acid.

Apart from the methods described above, the fact that ammonium lactate is directly esterified with an alcohol has been to the art from long ago (Industrial and Engineering Chemistry, Vol. 44, No. 9, pp. 2189-2191, Sept. 1952).

In order for this direct esterification with an alcohol to be actually applied effectively to the synthesis of a lactic ester or to the purification of lactic acid, however, numerous problems which, as found by US as will be specifically described afterward, are attendant on the use of the alcohol must be solved. When the esterification is effected simply by adding the alcohol without solving these problems, the lactic ester is formed at an unduly low yield hardly satisfactory from the commercial point of view.

Further in WO93/00440, Cockrem et al. propose a method which comprises adding butanol and sulfuric acid to a fermentation solution containing ammonium lactate, subjecting the resultant mixture to hydration and esterification thereby inducing crystallization of ammonium sulfate as a precipitate, separating the precipitate by filtration, and distilling the supernatant.

The literature makes a mention to the effect that the esterification of lactic acid with a higher alcohol having not less than 4 carbon atoms (such as, for example, butanol), as known already to the art, is advantageous in terms of process over the esterification using a lower alcohol because the dehydration is allowed to proceed by virtue of the azeotropy of the higher alcohol with water. Similarly, the practice of carrying out the esterification of lactic acid in the presence of such an acidic catalyst as sulfuric acid, acidic ammonium sulfate, or paratoluenesulfonic acid has been heretofore very much in vogue.

It is logically concluded, therefore, that this method is characterized by permitting the solution of butanol and butyl lactate in an organic solvent to precipitate ammonium sulfate in the form of relatively large crystals and, therefore, enjoy more satisfactory filtrability than the solution of the conventional method involving precipitation of gypsum and separation of the precipitate by filtration and allowing the lactic ester to be obtained with high purity at an amply satisfactory yield because the crystallization of ammonium sulfate proceeds simultaneously with the esterification.

This method, however, entails the disadvantage that the ammonia used in fermentation cannot be recycled through the fermentation because it is required to be wholly recovered in the form of ammonium sulfate and the operation of the method yields waste in a large amount.

The method is further at a disadvantage in augmenting the number of steps of process because the culture medium at the end of the esterification must be neutralized again and filtered to allow separation of a large amount of such solid substances as ammonium sulfate and the solid substances themselves must be deprived of butanol and other similar unwanted substances so as to be disposed of or put to some other uses.

In the light of the various problems mentioned above, this invention has for its object the provision of a novel method for the production of lactic acid and lactic esters.

Another object of this invention is to provide for the production of lactic acid or lactic esters by fermentation with a microorganism a method which, by the use of simple apparatus and procedure, permits easy manufacture of lactic acid possessing high purity and high optical activity to be attained in a high yield while lowering the cost involved in purification and decreasing the amount of by-products suffered to occur.

The objects of this invention are accomplished in a process involving microorganic fermentation of lactic acid by (1) a method for the production of a lactic ester characterized by (a) carrying out the adjustment of pH of a culture medium undergoing the fermentation of lactic acid with ammonia, (b) adding an alcohol having 4 or 5 carbon atoms to at least one solution selected from the group consisting of a solution of ammonium lactate obtained by the fermentation, the concentrate of the solution of ammonium lactate mentioned above, and the solution resulting from separation of solid substances including the microorganic cells used for the fermentation from either of the solutions mentioned above and heating the resultant mixture thereby inducing dehydration and esterification of lactic acid with the alcohol and, at the same time, effecting liberation and recovery of ammonia, and (c) adding a mineral acid to the solution obtained by the esterification reaction of (b) and heating and dehydrating the resultant mixture in an acidic state thereby promoting and completing the esterification of the lactic acid with the alcohol. The lactic ester can be easily separated from this esterification reaction solution and then purified.

The objects of this invention are further accomplished by (2) a method for the production of a lactic ester set forth in (1) above, wherein the amount of the alcohol to be added is 1.5 to 4 mols per mol of the lactic acid.

The objects of this invention are also accomplished by (3) a method for the production of a lactic ester set forth in (2) above, wherein the alcohol is n-butanol.

Further, the objects of this invention are accomplished by (4) a method for the production of lactic acid by the hydrolysis of the lactic ester produced by the method set forth in any of (1) through (3) above.

With a view to accomplishing the objects described above, we have continued a diligent study in search of a novel method for the production of lactic acid and lactic esters to find that when the adjustment of pH of a culture medium undergoing fermentation of lactic acid as with lactic acid bacteria is effected by the use of ammonia, (1) esterification of lactic acid with an alcohol having four or five carbon atoms is attained by directly adding the alcohol to a fermentation broth containing ammonium lactate and heating the resultant mixture, (2) the excess alcohol which occurs in the form of an azeotrope with water during the esterification mentioned above is easily refluxed to the site of reaction because the vapor of this azeotrope easily separates into two layers when cooled, (3) the greater part of ammonia is liberated simultaneously with the esterification of lactic acid with the alcohol and the liberated ammonia can be easily recovered, (4) the yield of synthesis of the lactic ester can be exalted by adding to the ammonia which remains after completion of the liberation of ammonia a slight access of a mineral acid thereby forming an acidic catalyst and continuing the heating of the fermentation broth and further decreasing the amount of a by-produced salt being formed, and (5) the lactic ester can be easily separated from the resultant reaction solution by distillation, the deposition of impurities to the inner walls of a reaction kettle occurs only sparingly during the distillation, the lactic ester can be easily refined to a high degree of purity by means of distillation, and the lactic acid can be easily obtained by hydrolyzing the lactic ester. The present invention has been perfected as a result.

Owing to the use of an alcohol having 4 or 5 carbon atoms in the production of lactic acid and lactic esters by the method of this invention, the distillate which is obtained when a mixed vapor of water and the alcohol during the course of the esterification separates into two layers composed of a water phase and an alcohol phase and, because of amply low mutual solubility of these two phases, the alcohol phase can be refluxed efficiently in its unmodified form to the site of reaction and the lactic ester produced meanwhile can be easily refined by the conventional technique of distillation. Thus, the method of this invention proves advantageous from the commercial point of view.

When the esterification is effected simply by adding an alcohol to the ammonium lactate solution, the lactic ester is produced in an unduly low yield. The addition of such a mineral acid as sulfuric acid or hydrochloric acid serves the purpose of promoting the esterification to the extent of enabling the lactic ester to be produced in a yield fully satisfactory from the commercial point of view.

The present invention manifests the following peculiar effects as compared with the conventional method.
(1) The esterification does not need to be preceded by a step of liberating lactic acid from a lactate and separating the lactic acid in a crude form.
(2) The greater part of ammonia used as a neutralizing agent for the fermentation of lactic acid can be separated and recovered and consequently recycled through the fermentation.
(3) In consequence of the advantage obtained in (2) above, the amount of a by-produced salt can be decreased notably as compared with the conventional method.
(4) The esterification of lactic acid simultaneously proceeds with the separation of ammonia. By causing the alcohol separated by distillation to be refluxed to the reaction system and consequently keeping the reaction system in the state of an excess of alcohol as described above, the esterification and the recovery of ammonia can be easily promoted.
(5) By further promoting alcoholysis with an added mineral acid, the lactic ester is formed in a high yield and separated from the components of a culture medium by distillation and obtained in a highly refined state by the use of a simple apparatus.
(6) By a method which uses a relatively clear culture medium for the fermentation, a method which removes solid substances in advance of a pretreatment of a culture medium by separation with a membrane, a method which carries out fermentation while separating a culture medium and bacterial cells with a membrane during the course of the fermentation, or a method which removes bacterial cells and other solid substances after the fermentation, the distillation can be carried out directly without preparatorily separating ammonium sulfate or acidic ammonium sulfate and other precipitates which occur within the stage for promoting the esterification up to the site of esterification.
(7) Further during the separation by distillation of the lactic ester and an excess of alcohol from the esterification solution, part of such residual liquid-phase components as acidic ammonium sulfate can be recycled through the stage for promotion of the esterification up to the site of esterification. This recycle aids in enhancing the yield of the lactic ester.
(8) From the lactic ester obtained in any of (1) to (7) mentioned above, lactic acid of high purity can be easily obtained by hydrolyzing the lactic ester.
(9) The process can be easily designed for a continuous operation.

Fig. 1 is a schematic diagram illustrating an apparatus for the production of butyl lactate by a method involving addition of n-butanol to the concentrate of ammonium lactate obtained by fermentation, as one embodiment of this invention directed to the production of a lactic ester.

Fig. 2 is a schematic diagram illustrating a device for the removal of ammonia used in one working example of this invention.

Fig. 3 is a schematic diagram illustrating a device for the separation of butyl lactate by distillation in the working example of this invention.

Fig. 4 is a schematic diagram illustrating a device for the hydrolysis of butyl lactate in the working example of this invention.

Fig. 5 is a schematic diagram illustrating a device for continuous removal of ammonia in the working example of this invention.

Now, this invention will be described in detail below.

The fermentation of lactic acid with a microorganism in accordance with this invention is not discriminated on account of the particular type of a method to be employed for the fermentation. For example, a method which effects the fermentation by preparing a culture medium using sucrose, lactose, glucose, and/or starch as a main raw material further incorporating therein such nutrients as yeast extract and corn steep liquor for lactic acid bacteria and inorganic salts necessary for fermentation, and inoculating to the culture medium such so-called lactic acid bacteria as Lactobacillus or Lactococcus or microorganic cells such as of Rhizopus oryzae which possess an ability to ferment lactic acid can be adopted.

As the first step in the process of this invention including the fermentation of lactic acid with a microorganism, the step of fermentation so to speak which comprises adjusting the pH value of the culture medium during the course of fermentation by the use of aqua ammonia in proportion to the amount of lactic acid being formed by the fermentation is carried out.

As the ammonia to be used in the step of fermentation, ammonia gas and aqua ammonia can be used without reference to the state of aggregation (gaseous, liquid, etc.). It is, however, preferable to use aqua ammonia which allows easy handling. When aqua ammonia is used, though the concentration thereof is not particularly restricted, it is generally in the range of 20 to 30%. When the fermentation relies on the bacterial cells of Rhizopus oryzae which possess an ability to ferment lactic acid, the liquid culture medium being used for the fermentation must be aerated because the bacteria are aerobic in nature. The ammonia gas can be blown into the culture medium with the air for aeration mentioned-above, for the sake of attaining the pH adjustment.

The pH of the culture medium during the fermentation of lactic acid in the step of fermentation is varied with the kind of microorganism and the conditions of fermentation and is decided freely depending on the various conditions involved in the operation of fermentation. Generally, the pH is adjusted in the range of 4 to 7, preferably 5.5 to 6.5. If the pH is less than 4, the fermentation proves undesirable because the rate of fermentation is lowered and the microorganisms being used in the fermentation eventually become extinct. The pH value does not need to be kept at a fixed level at all times. In the case of a culture medium using bacterial cells of Rhizopus oryzae which possess an ability to ferment lactic acid, for example, the pH of this culture medium may be controlled to the optimum level for the culture of the bacterial cells. During the fixed period in which the bacterial cells form pellets, the pH value of this culture medium may be kept at a slightly lower level because the pellets are preferable to be formed in generally uniform shape and size. Thus, the pH value can be freely adjusted.

As the second step in the process of this invention including the fermentation of lactic acid with a microorganism, the step of removal of ammonia so to speak in which the dehydration and the esterification of lactic acid with an alcohol having four or five carbon atoms are effected simultaneously with the liberation of ammonia for recovery by adding the alcohol to at least one solution selected from the group consisting of an ammonium lactate solution obtained by fermentation, the concentrate of the ammonium lactate solution, and a solution resulting from removal from either of the solutions mentioned above of solid substances including bacterial cells used in the fermentation and heating the resultant mixture is carried out.

The ammonium lactate solution which is obtained by the fermentation in the step of removal of ammonia contains the microorganisms (bacterial cells) used for the fermentation, residues of the nutrient or inorganic salt, and the portions of such raw materials as saccharine material and starch which have escaped assimilation in the fermentation in addition to the ammonium lactate included in the culture medium at the end of the fermentation. These substances so contained in the ammonium lactate solution are not required to be separated from the culture medium before the process is advanced to the next step except when the microorganism used in the fermentation happens to be that of Rhizopus oryzae which forms bacterial cells in a particularly large amount. They can be directly used as part of the ammonium lactate solution.

Preferably, a concentrated ammonium lactate solution is used in the place of the ammonium lactate solution. This is because the ammonium lactate solution obtained by the fermentation in the step of removal of ammonia generally has a low ammonium lactate concentration below 15% by weight at the end of fermentation. Generally, the ammonium lactate solution is concentrated before it is subjected to esterification with an added alcohol. Though the extent to which the ammonium lactate solution is concentrated is arbitrary, it is generally proper in the range of 60 to 75% by weight, preferably 65 to 70% by weight as reduced to lactic acid in the concentrated solution. Though the conditions for this concentration do not need to be particularly restricted, the concentration is generally desired to be carried out under normal pressure at an elevated temperature in the range of 105° to 140°C, preferably 120° to 125°C, for the purpose of allowing water to vaporize and, at the same time, permitting a small amount of ammonia being liberated during the course of concentration to be easily condensed and collected.

By this operation of concentration, the water and ammonia being vaporized can be collected and circulated to be reclaimed respectively as the water for the preparation of the culture medium for fermentation and as the ammonia for the adjustment of pH during the course of fermentation.

It is desirable to use the solution which is obtained by removing solid substances including the microorganism used for the fermentation from the ammonium lactate solution or the concentrate of the ammonium lactate solution in the place of the ammonium lactate solution as occasion demands. This is because the subsequent step necessitates a treatment for separation of bacterial cells by precision filtration and a treatment for separation of the residues of other polymers by ultrafiltration when the adhesion of residues of fermentation to the inner walls of an esterification reaction vessel is so serious as to pose a problem in the subsequent step and because the fermentation which happens to use such auxiliary raw materials as corn steep liquor requires a treatment for removal of unnecessary solid substances contained in the auxiliary raw material to be performed prior to the esterification induced by addition of an alcohol to the culture medium for fermentation.

The separation of the solid substances containing the microorganism used for the fermentation can be effectively carried out either before or after the concentration of the fermentation solution described above.

Then, in the step for removal of ammonium, an alcohol having 4 or 5 carbon atoms (hereinafter referred to simply as "C₄-C₅ alcohol") is added to at least one solution selected from the group consisting of the ammonium lactate solution obtained by the fermentation mentioned above, the concentrate of this ammonium lactate solution, or the solution resulting from removal from either of the solutions mentioned above of solid substances including the microorganism used for the fermentation (hereinafter referred to simply as "ammonium lactate solution") and the resultant solution is heated to effect the dehydration of this ammonium lactate solution and the esterification of lactic acid with the alcohol simultaneously with the liberation of ammonia for recovery.

The C₄ - C₅ alcohols which are effectively usable herein include n-butanol, isobutanol, t-butanol, n-amyl alcohol, sec-amyl alcohol, t-amyl alcohol, isoamyl alcohol, sec-isoamyl alcohol, active amyl alcohol, diethyl carbinol, and t-butyl carbinol, for example. Among other C₄ - C₅ alcohols cited above, n-butanol proves particularly preferable. When n-butanol is adopted, the amount of the alcohol to be added as specifically described afterward is in the range of 1 to 10 mols, preferably 1.5 to 4 mols, per mol of the lactic acid (including lactates and polymers of lactic acid) contained in the ammonium lactate solution mentioned above. Then, the heating of the resultant mixture is carried out under normal pressure at an elevated temperature in the range of 100° to 170°C, preferably 120° to 150°C. By using this heat treatment for simultaneously effecting the esterification and the liberation of ammonia for recovery, the ratio of recovery of ammonia in the case of using the concentrate of the ammonium lactate solution can be easily made to reach a level exceeding 90%, including the amount of ammonia liberated during the course of concentration, as shown in the working example to be cited afterward. In this case, the n-butanol is fated to form an azeotrope with water. Since this azeotropic composition separates into the two phases of n-butanol and water when it is suitably cooled and collected, the reflux of n-butanol to the esterification reaction system can be easily accomplished. Thus, n-butanol proves an extremely desirable alcohol.

In the case of an alcohol having more than 5 carbon atoms, though liberation of ammonia by esterification is obtained, the subsequent formation of a lactic ester by distillation is attained only with difficulty because the formed lactic ester has a high boiling point. In the case of ethanol or methanol which has a low boiling point, ammonia cannot be liberated satisfactorily because the reaction temperature is not elevated. In the case of an alcohol having three carbon atoms, no efficient operation can be obtained because the phase separation of this alcohol from water does not proceed fully.

The amount of the alcohol to be added for use in the present invention is preferable to be in the range of 1 to 10 mols, preferably 1.5 to 4 mols, per mol of the lactic acid (including lactates and polymers of lactic acid) which is contained in the ammonium lactate solution mentioned above.

The conditions under which the esterification is carried out in the step for removal of ammonia are variable with the kind of alcohol to be used for the esterification. The reaction pressure is not restricted in any sense. The esterification proceeds fully satisfactorily under normal pressure. Though the temperature conditions used for the batchwise operation and those used for the continuous operation are different, the reaction temperature is generally desired to be in the range of 100° to 170°C, preferably 120° to 150°C. If this temperature is lower than 120°C, the esterification is deficient in productivity because the liberation of ammonia proceeds at an unduly low rate. If the temperature is lower than 100°C, the reaction fails to proceed at an economically satisfactory rate. If the temperature exceeds 150°C, such adverse phenomena as partial decomposition of lactic acid and formation of a corresponding ether formed of two alcohol molecules appear. If the temperature exceeds 170°C, the phenomena of decomposition of lactic acid and formation of ether of two alcohols occur more conspicuously.

As respects the simultaneous liberation of ammonia for recovery with the esterification mentioned above, since the vapor generated in consequence of the esterification contains ammonia, water, and alcohol, the reaction vessel to be used for the esterification is provided with stirring vanes and furnished thereover with a multistage distillation column for the purpose of precluding otherwise possible distillation of the lactic ester, the distillation column is provided at the outlet thereof with a condenser for the purpose of cooling and collecting water and alcohol, the condenser is provided thereunder with a tank capable of separation of water and alcohol for the purpose of refluxing the alcohol to the reaction vessel, and the reaction vessel is furnished with a water reservoir for the purpose of cooling water and allowing the cooled water to absorb the portion of ammonia gas which has escaped being collected by the condenser mentioned above. This invention does not need to be limited to the method described above but may be worked by a suitably selected method.

When the step for removal of ammonia mentioned above is performed batchwise, the ammonium lactate solution contains a large amount of water at first. When the culture medium for fermentation which contains the ammonium lactate solution so abounding in water as mentioned above is kept at an elevated temperature, the inner wall of the reaction vessel collects scale though to a slight extent. When the step is continuously performed and the ammonium lactate solution in the reaction vessel is kept in a state substantially devoid of water, the inner wall of the reaction vessel collects virtually no scale notwithstanding the ammonium lactate solution separates solid substances. The step for removal of ammonium, therefore, is desired to be performed continuously rather than batchwise.

The batchwise operation mentioned above suffers the reaction temperature to rise gradually in consequence of the advance of the removal of ammonia and the dehydration in the step of removal of ammonia. In contrast thereto, the continuous operation can be expected to allow a decrease in the average retention time and a reduction in the size of apparatus because the reaction temperature is kept constantly at an elevated level.

As the third step in the process of this invention including the fermentation of lactic acid with a microorganism, the step for promotion of the esterification so to speak in which the esterification of lactic acid with the alcohol is promoted and completed for the purpose of obtaining the lactic ester desired by adding a mineral acid to the solution resulting from the step for removal of ammonia mentioned above and heating to dehydrate the resultant mixture in an acidic state is carried out.

In the step for removal of ammonia mentioned above, part of the lactic acid undergoes self-condensation. In the step for promotion of the esterification, therefore, the ratio of formation of the lactic ester is barely in the range of 50 to 80% by weight at most based on the amount of the lactic acid (including lactates and polymers of lactic acid) contained in the ammonium lactate solution mentioned above, notwithstanding the ratio of recovery of ammonia reaches a level exceeding 90%. This ratio of formation of the lactic ester is not sufficient for the purpose of obtaining the lactic ester by purification resorting to direct distillation.

As a step for promoting the esterification, therefore, this invention attains the promotion of the esterification of the remaining lactic acid and consequently exalts the yield of the lactic ester by adding to the reaction mixture a mineral acid in an amount of not less than the equivalent weight, preferably the equimolar weight, relative to the remaining ammonia thereby forming an acidic catalyst and continuing the thermal dehydration of the lactic acid. Though the amount of the mineral acid to be added as an acidic catalyst has no upper limit in particular, it is preferable to be small from the standpoint of economy. It is required to be suitably varied depending on the amount of the remaining ammonia and the component incorporated as a buffer in the culture medium. In the culture medium of a common run, the mineral acid added in an amount at least enough to neutralize the remaining ammonia and not more than 0.2 part per part of the lactic acid suffices for the purpose.

The mineral acid to be used in the step for promoting the esterification is not particularly restricted. It is most advantageous to use sulfuric acid from the economic point of view.

Since the greater part of ammonia has been already recovered in the preceding step for removal of ammonia, the amount of a salt suffered to be by-produced in the present step for promoting the esterification is far smaller than in the equivalent step of the conventional method.

In the present step for promoting the esterification, the esterification proceeds sufficiently even at a lower temperature than in the preceding step for removal of ammonia. For the sake of exalting the final yield of the lactic ester, therefore, it is allowable to add an alcohol to the reaction mixture so as to shift the equilibrium of the esterification toward the formation of the lactic ester. The alcohol to be added in this case may be any of the alcohols having four or five carbon atoms. This alcohol can be used in its unmodified form. Among other alcohols answering the description, n-butanol proves particularly desirable.

The conditions to be used for the reaction in the step for promoting the esterification are variable with such factors as the kind of the alcohol to be used. The reaction pressure has no restriction of any sort. Generally, normal pressure suffices to promote the esterification as aimed at. For the reason given below, it is allowable to use a reduced pressure in the range of 100 to 760 mmHg, preferably 200 to 350 mmHg, for the reaction. When the esterification is promoted under normal pressure, the reaction temperature reaches a level approximating 130°C as specifically described afterward and the conversion of every two molecules of the added C₄ - C₅ alcohol into a corresponding ether molecule (for example, n-butanol giving rise to dibutyl ether) occurs copiously. This phenomenon results directly in a decrease in the amount of the C₄ - C₅ alcohol because the ether does not easily decompose. Further, the recycled use of the alcohol is fated to require extra time and labor for the separation of the ether which continues to accumulate with the advance of the reaction. For the purpose of precluding such occurrence of the ether as mentioned above, the esterification can be promoted at such a relatively low temperature as shown hereinbelow under a reduced pressure falling in the range mentioned above. Then, the reaction temperature is generally in the range of 100° to 160°C, preferably 120° to 130°C when the reaction is performed under normal pressure and not under the reduced pressure mentioned above. Under the reduced pressure, the reaction is generally desired to be carried out at a temperature in the range of 60° to 120°C, preferably 90° to 110°C.

Further, in the process for promoting the esterification, the alcohol begins to separate in the form of an azeotrope with water in consequence of the dehydration. The azeotrope thus formed, similarly to that which occurs in the step for removal of ammonia mentioned above, is preferable to be separated into the alcohol and water, with the separated alcohol refluxed to the site of esterification in the step for promoting the esterification. This measure proves excellent even from the economic point of view because it permits effective reclamation of the alcohol.

The esterification solution in which the esterification has been thoroughly promoted in the step for promoting the esterification contains the alcohol, the lactic ester of the alcohol, the mineral acid salt of ammonia, the microorganic cells of lactic acid bacteria, and other impurities and virtually no water. Such solid substances as the ammonia salt, the polymer impurities, and the lactic acid bacteria remain as solid components and assume the state of slurry.

The method to be adopted for the separation of the lactic ester and the excess of alcohol from the esterification solution has no restriction in particular. This separation can be easily carried out batchwise or continuously by the conventional technique of distillation. The esterification solution, for example, may be distilled in its unmodified state under a reduced pressure. When the viscosity of the residue which exists in the esterification vessel during the course of the distillation is unduly high or when the deposition of scale on the inner wall of the vessel is conspicuous, the distillation may be carried out after the solid substances mentioned above have been preparatorily removed as by filtration through a membrane or by decantation. Preferably, the distillation is performed after crystalline solid components of such salts as ammonium sulfate and ammonium hydrogen sulfate have been exclusively separated by filtration of the conventional method. In this case, microorganic cells such as of lactic acid bacteria and other minute particles which are suffered to remain in the esterification solution instead of being separated by filtration pose no problem at all and the substances remaining on the inner wall of the reaction vessel after the distillation exhibit excellent flowability. Further, the inner wall of the reaction vessel shows absolutely no sign of adhesion of any matter.

When the method which uses a relatively clear culture medium for fermentation, the method which gives a pretreatment of separation with a membrane to a culture medium so as to effect preliminary removal of solid substances from the culture medium, the method which effects separation of a culture medium and microorganic cells by means of a membrane while the fermentation is in process, or the method which effects removal of microorganic cells and other solid substances at the end of fermentation is adopted, the separation of the lactic ester and the excess of alcohol from the esterification solution can be attained by directly distilling the esterification solution without separating therefrom the ammonium sulfate or acidic ammonium sulfate and other precipitates which occur within the step for promoting the esterification up to the site of esterification.

In the distillation part for separating the lactic ester and the excess of alcohol from the esterification solution, part of the residues of such liquid-phase components as acidic ammonium sulfate can be recycled through the site of esterification in the step for promoting the esterification. By this recycling, the yield of the lactic ester can be further improved.

Optionally, the lactic ester thus obtained may be subjected repeatedly to the conventional treatment of distillation to be finished in the form of highly purified lactic ester.

Then, the method of this invention for the production of lactic acid resides in causing the lactic ester which has been obtained by the method described above to be hydrolyzed in the presence of an acid catalyst of ordinary run. In consequence of this hydrolysis, the alcohol remaining in the lactic ester can be removed and the lactic acid can be obtained in a state refined to a high degree of purification.

As typical examples of the acid catalyst which is usable effectively for this hydrolysis, ion-exchange resins and mineral acids may be cited.

Now, the method of this invention for the production of a lactic ester will be described more specifically below with reference to the accompanying drawings.

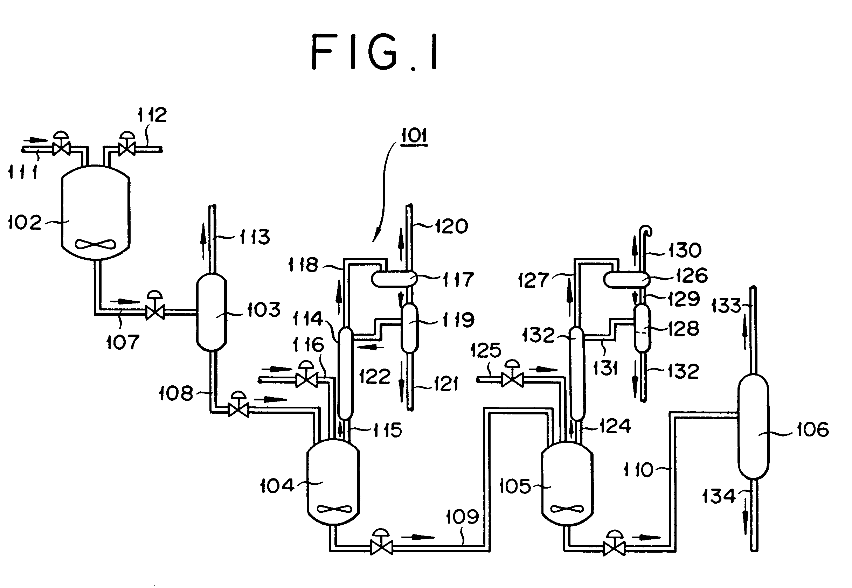

Fig. 1 is a schematic diagram illustrating an apparatus for the production of butyl lactate used for the production of butyl lactate by the addition of n-butanol to the concentrate of ammonium lactate obtained by fermentation with a microorganism as one embodiment of this invention in the production of a lactic ester.

An apparatus 101 for the production of butyl lactate, as illustrated in Fig. 1, is constructed by connecting a fermentation column 102, a concentrator 103, an ammonia removing can 104, an esterification promoting can 105, and a flush vaporizing part 106 with relevant pipes 107, 108, 109, and 110.

To the fermentation column 102, a raw material column (not shown) and an ammonia storage column (not shown) are connected respectively via a pipe 111 and a pipe 112.

Then, to the concentrator 103, a water-ammonia collection column (not shown) is connected via a pipe 113.

To the ammonia removing can 104, a distillation column 114 and an alcohol storage column (not shown) are connected via a pipe 115 and a pipe 116. A condenser 117 is connected via a pipe 118 to the distillation column 114. The condenser 117 is connected to an ammonia absorber (not shown) and a two liquid phase separation can 119 via a pipe 120 and a pipe 121. The two liquid phase separation can 119 is connected to the top part of the distillation column 114 via a pipe 122 so as to reflux the upper phase component of the two liquid phase separation can 119 and then connected to a water recovery column (not shown) via the pipe 121 so as to recover the lower phase component of the two liquid phase separation can 119.

Further, to the esterification promoting can 105 mentioned above, a distillation column 123 and a mineral acid storage column (not shown) are connected via a pipe 124 and a pipe 125. A condenser 126 is connected via a pipe 127 to the distillation column 123. A two liquid phase separation can 128 is connected to the condenser 126 via a pipe 129. The condenser 126 is provided with a discharge tube 130 opening into the ambient air. The two liquid phase separation can 128 mentioned above is connected to the top part of the distillation column 123 via a pipe 131 so as to reflux the upper phase component of the two liquid phase separation can 128 and then connected to a water recovery column (not shown) via a pipe 132 to as to recover the lower phase component of the two liquid phase separation can 128.

The flush vaporizing part 106 is connected to a butyl lactate recovery column (not shown) and a residue recovery column (not shown) respectively via a pipe 133 and a pipe 134.

The pipes mentioned above are severally provided, when necessary, with valves (not shown) adapted to control flows of raw materials and reaction solutions to reaction vessels (such as, for example, the fermentation column 102, concentrator 103, ammonia removing can 104, esterification promoting can 105, and flush vaporizing part 106).

The production of butyl lactate by the use of the apparatus 101 for the production of butyl lactate constructed as described above, when lactic acid bacteria are selected as a microorganism for fermentation, is accomplished by supplying a raw material prepared by inoculating the lactic acid bacteria to a culture medium composition for fermentation comprising such nutrients as glucose and corn steep liquor from the raw material column via the pipe 111 to the fermentation column 102, keeping the raw material in the fermentation column 103 at an optimum temperature for fermentation of lactic acid in the range of 30° to 50°C, properly stirring the raw material in an atmosphere fit for the microorganic fermentation, e.g. an anaerobic atmosphere for lactic acid bacteria, and simultaneously feeding 20 to 30% aqua ammonia from the ammonia storage column via the pipe 112 to the stirred raw material thereby controlling the raw material at a pH in the range of 5.5 to 6.5 to effect the fermentation. The yield of lactic acid based on the amount of glucose in the step of fermentation is variable with the species of a microorganism to be used. It is in the approximate range of 94 to 97% when the fermentation uses lactic acid bacteria.

Subsequently, the fermentation solution which is formed in the fermentation column 102 is forwarded via the pipe 107 to the concentrator 103, dehydrated therein by boiling under normal pressure, and concentrated generally to a lactic acid concentration in the range of 65 to 70%. The distilled water and partially liberated ammonia are transferred via the pipe 113 to the water-ammonia collecting column, wherein the water is boiled for through removal of the ammonia. The ammonia thus expelled is recovered by being absorbed in a collection water prepared separately and kept in a cooled state.

Then, the concentrate obtained in the concentrator 103 and having a lactic acid concentration in the range of 65 to 70% is forwarded via the pipe 108 to the ammonia removing can 104. From the alcohol storage column, n-butanol is fed generally in an amount in the range of 1.5 to 4 mols per mol of lactic acid via the pipe 116 to the concentrate to prepare a reaction solution. The reaction solution thus prepared in the ammonia removing can 104 is stirred with stirring vanes provided in the can 104 and, at the same time, heated to a temperature in the range of 120° to 150°C to induce esterification of the lactic acid with n-butanol and promote the esterification. The azeotrope of water with n-butanol which is produced by the thermal reaction is collected via the distillation column 114 disposed above the ammonia removing can 104 in the condenser 117 disposed above the top of the distillation column 114. The collected azeotrope is separated into a water phase and a butanol phase in the two liquid phase separating can 119 disposed below the condenser 117. The n-butanol as the upper phase component (containing a small amount of water) is refluxed via the pipe 121 to the upper part of the distillation column 114 and the water as the lower phase component (including a small amount of n-butanol and ammonia) is extracted from time to time via the pipe 122 and recovered in the water recovery column. The ammonia gas which has passed through the distillation column 114 is not collected in the condenser 117 but is forwarded via the pipe 120 and collected in a water-cooled ammonia absorber.

Subsequently, the solution which has undergone the reaction in the ammonia removing can 104 (the whole lactic acid component containing ester, salt, etc.) is forwarded via the pipe 109 to the esterification promoting can 105. From the mineral acid storage column, concentrated sulfuric acid is fed generally in an amount in the range of 0.05 to 0.2 mol per mol of lactic acid via the pipe 125 to the solution in the esterification promoting can 105 to prepare a reaction solution. The reaction solution thus prepared in the esterification promoting can 105 is stirred with stirring vanes provided in the can 105 and, at the same time, heated continuously at a temperature in the range of 120° to 130°C to promote the esterification. The azeotrope of water with n-butanol which is produced by the thermal reaction is forwarded via the distillation column 123 disposed above the esterification promoting can 205 and collected in the condenser 126 disposed above the top of the distillation column 123 and separated into a water phase and a butanol phase in the two liquid phase separation can 128 disposed below the condenser 126. The n-butanol as the upper phase component (containing a small amount of water) is refluxed via the pipe 131 to the upper part of the distillation column 123 and the water as the lower phase component (containing a small amount of n-butanol) is extracted from time to time via the pipe 132 and recovered in the water recovery column. An extremely small amount of ammonia gas escapes being collected in the condenser 126 and released through the discharge tube 130 into the ambient air.

Finally, the solution which has undergone the reaction in the esterification promoting can 105 is forwarded via the pipe 110 to the flush vaporizing part 106. Subsequently, the solution now held in the flush vaporizing part 106 is kept in an equilibrated state at a temperature in the range of 100° to 150°C under a pressure in the range of 10 to 100 torrs and separated into a liquid phase and a gaseous phase. Subsequently, the acidic ammonium sulfate and other residues as liquid phase components are forwarded via the pipe 134 and recovered in the residue recovery column and the butyl lactate and n-butanol as gaseous phase components are forwarded via the pipe 133 and collected in the butyl lactate recovery column. Thus, the butyl lactate aimed at is obtained. Then, such solid substances as acidic ammonium sulfate present in the liquid phase mentioned above may be removed before they reach the flush vaporizing part 106 by means of a filter adapted to separate such solid substances and inserted in the path of the pipe 110.

Now, the present invention will be described below with reference to working examples.

In the following examples, the lactic acid content and the glucose content of the fermentation solutions were invariably determined by high-speed liquid chromatography (differential refractance detector). The reaction solutions were analyzed for butyl lactate and dibutyl ether by gas chromatography. They were analyzed for ammonia content by high-performance liquid chromatography (fluorescent detector).

### Example 1

### (1) Concentration of culture medium for fermentation

A culture medium composition for fermentation prepared by diluting 100 g of glucose and 40 g of corn steep liquor with tap water to a total volume of 1 liter was placed in a jar fermenter and sterilized by a heat treatment at 121°C for 15 minutes. The sterilized culture medium composition and 40 ml of the cells of the microorganism, Lactobacillus casei subsp. rhamnosus IFO 3863 inoculated thereto were kept at 42°C, stirred at 50 rpm, sparged with a small amount of nitrogen, and controlled to pH 6.0 with 10 mol% aqua ammonia.

The fermentation lasted for 55 hours. The yield of lactic acid based on glucose was 96.2%.

Part of the fermentation broth (1.02 mols as reduced to lactic acid) was transferred into a round-bottomed flask, boiled therein under normal pressure to be dehydrated to a lactic acid concentration of 62.0%. The distilled water (containing partially liberated ammonia) was collected and boiled for thorough expulsion of ammonia. The ammonia thus removed was absorbed as cooled in the water which was separately prepared for the purpose of collecting ammonia. The amount of the water thus collected was 8.22 g and that of ammonia collected was 0.135 mol.

### (2) Batchwise removal of ammonia

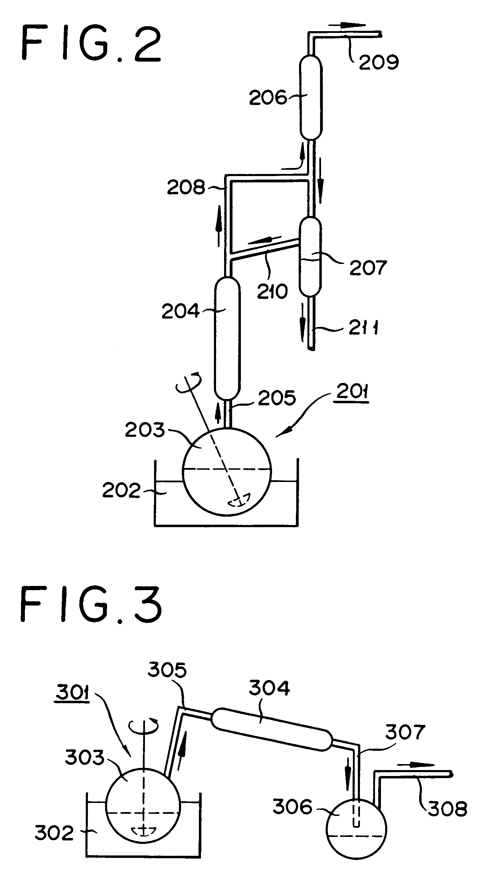

Fig. 2 is a schematic diagram illustrating an apparatus used in the present example for removal of ammonia.

An apparatus 201 for the removal of ammonia, as illustrated in Fig. 2, is provided with an oil bath 202 and a reaction vessel 203 furnished with stirring vanes and immersed in the oil of the oil bath 202. To the reaction vessel 201, a distillation column 204 is connected via a pipe 205. The distillation column 204 is connected via a forked pipe 208 to a condenser 206 and a two phase separator 207. The condenser 206 is connected via a pipe 209 to an ammonia absorber (not shown). The two phase separator 207 is connected via a pipe 210 to the forked pipe 208 so as to reflux the upper phase component of the two phase separator 207 and further connected via a pipe 211 to a water recovery column (not shown) so as to recover the lower phase component of the two phase separator 207.

The concentrate obtained by concentrating the culture medium for fermentation mentioned in (1) above to a lactic acid concentration of 62.0% was esterified and, at the same time, subjected to removal of ammonia by the use of the apparatus 201 for removal of ammonia constructed as described above.

First, in the reaction vessel 203, the concentrate mentioned above (1.02 mols as lactic acid) and 2.55 mols of n-butanol (2.5 mols per mol of lactic acid) were combined to form a reaction solution. The reaction solution in the reaction vessel 203 was heated and, at the same time, stirred with the stirring vanes provided in the reaction vessel 203 to induce a reaction. This heat treatment was carried out at a temperature (of the oil in the oil bath 202) in the range of 150° to 160°C. The azeotrope of n-butanol with water which was formed by the heat treatment was collected by the condenser 206 disposed above the top of the distillation column 204 which was installed above the reaction vessel 203 and separated into a water phase and a butanol phase in the two phase separator 207 disposed below the condenser 206. The butanol as the upper phase component (containing a small amount of water) was refluxed via the pipe 210 to the forked pipe 208 disposed above the distillation column 204. The water as the lower phase component (containing a small amount of n-butanol) was extracted from time to time via the pipe 211 and recovered in the water recovery column. The ammonia gas which was vaporized was collected by the use of an ammonia absorber (ice water) because it escaped being collected by the condenser 206.

The temperature of the reaction solution inside the reaction vessel 203 gradually rose after the start of the reaction and reached 132°C at the end of 10 hours of the reaction. Then, this reaction was stopped because the distillation of water through the top of the distillation column 204 substantially ceased. The reaction solution which remained inside the reaction vessel 203 after the stop of the reaction contained 0.784 mol of butyl lactate and 0.0612 mol of ammonia.

Inside the reaction vessel 203 after the esterification, solid substances were found adhering copiously to the part of the inner wall near the level of the reaction solution. They were also found adhering, though slightly, to the part of the inner wall below the level of the reaction solution.

### (3) Promotion of esterification

The reaction solution (1.00 mol as whole lactic acid including ester and salt) which remained in the reaction vessel 203 mentioned above after a portion thereof had been extracted as a sample for analysis was combined with 0.08 mol of concentrated sulfuric acid and 0.5 mol of n-butanol and thermally dehydrated continuously to promote the esterification under the same heating conditions as used for the batchwise removal of ammonia indicated in (2) above. After the esterification had lasted for 3 hours, the reaction was stopped because the distillation of water through the top of the distillation column 204 substantially ceased. The reaction solution which remained inside the reaction vessel 203 after the stop of the reaction contained minute particles of solid substances. None of such solid substance was found adhering to the inner wall of the reaction vessel 203. The reaction solution contained 0.987 mol of butyl lactate.

### (4) Distillation of butyl lactate

Then, the reaction solution produced by the esterification (containing 0.96 mol of whole lactic acid after the sampling for analysis) was filtered to remove solid substances and obtain a filtrate. The solid substances were washed with n-butanol. The washings were combined with the filtrate mentioned above to form a reaction solution for distillation of butyl lactate.

Fig. 3 is a schematic diagram illustrating an apparatus for distillation of butyl lactate which was used in the present example.

An apparatus 301 for distillation of butyl lactate, as shown in Fig. 3, was provided with an oil bath 302 and a distillation can 303 furnished with stirring vanes and immersed in the oil of the oil bath 302. To the distillation can 303 is connected a condenser 304 via a pipe 305. The condenser 304 is connected via a pipe 307 to a receptacle 306. The receptacle 306 is connected to a vacuum pump (not shown) via a flexible tube 308. The distillation can 303 is connected to a distillate reaction solution storage column (not shown) via a pipe 309 so as to allow gradual supply of the distillate of the butyl lactate.

From the reaction solution produced by the esterification, butyl lactate was distilled by the use of the apparatus 301 for the distillation of butyl lactate constructed as described above.

First, the vacuum pump was set operating to vacuumize the whole body of the apparatus 301 for distillation and, at the same time, the temperature of the oil of the oil bath 302 was adjusted to keep the temperature of the reaction solution contained in the distillation can 303 in the neighborhood of 120°C and the reaction solution for distillation (composed of n-butanol and butyl lactate) was gradually forwarded from the storage column via the pipe 309 into the distillation column 303 and, in the meantime, the reaction solution was distilled by the use of the apparatus 301 for distillation. The amount of refined butyl lactate collected in the receptacle 306 in consequence of the treatment of distillation described above was 0.938 mol. The yield of distillation was 99.0%.

The residue in the distillation column 303 assumed high viscosity when cooled to normal room temperature. At a temperature above 60°C, it showed ample flowability. Absolutely no solid substance was found adhering to the inner wall of the distillation can 303.

It is clearly noted from the results of test described thus far that the separation of butyl lactate and n-butanol and the purification of butyl lactate could be easily carried out by such an ordinary treatment of distillation as used in the present working example.

Such high boiling components as polymer of lactic acid which consequently remained in the distillation can 303 were returned to the step for promoting the esterification indicated in (3) above, in which the polymer was thermally decomposed and utilized for the formation of a relevant lactic ester. Thus, the yield of the lactic ester could be exalted.

### Example 2

### Hydrolysis of butyl lactate

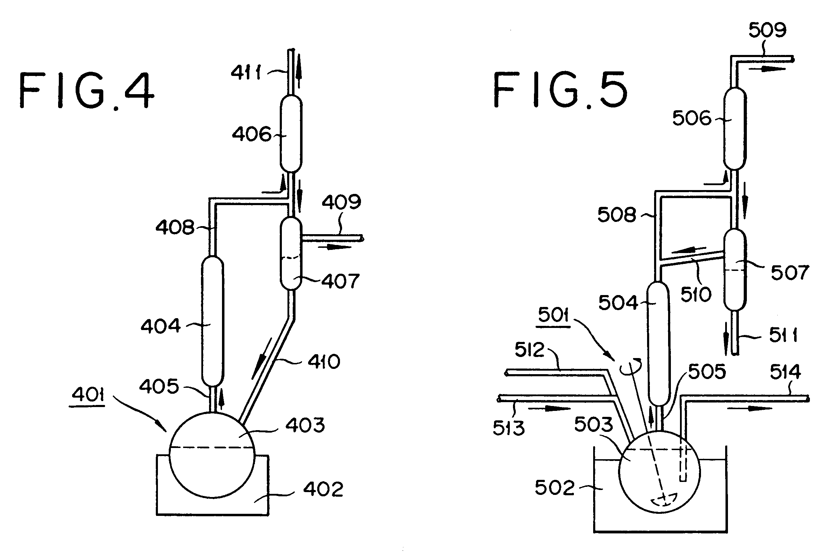

Fig. 4 is a schematic diagram illustrating an apparatus for hydrolysis of butyl lactate which was used in the present working example.

An apparatus 401 for hydrolysis of butyl lactate, as shown in Fig. 4, is provided with a mantle heater 402 and a hydrolyzing can 403 disposed on the mantle heater 402. A distillation column 404 is connected via a pipe 405 to the hydrolyzing can 403 and the distillation column 404 is connected via a forked pipe 408 to a condenser 406 and a two phase separation part 407. The two phase separation part 407 is connected via a pipe 409 to a butanol collecting column (not shown) so as to recover the upper phase component of the two phase separation part 407 and via a pipe 410 to the hydrolyzing can 403 so as to reflux the lower phase component of the two phase separation part 407. To the condenser 406 is connected a discharge tube 411 which opens into the ambient air.

The apparatus 401 for hydrolysis of butyl lactate which was constructed as described above was operated to hydrolyze the butyl lactate mentioned above and formed lactic acid.

The butyl lactate obtained in Example 1 and water were placed in the hydrolyzing can 403 and a strongly acidic cation-exchange resin (produced by Japan Organo Co., Ltd. and marketed under trademark designation of "Amberlite 200C") was added as an acid catalyst thereto. The resultant mixture in the hydrolyzing can 403 was heated at a temperature in the range of 95° to 110°C. The n-butanol which was generated by the ensuant hydrolysis formed an azeotrope with water and this azeotrope was collected in the condenser 405 disposed above the top of the distillation column 404. In the two phase separation part 407 disposed below the condenser 406, the azeotrope was separated into a water phase and an n-butanol phase. The n-butanol as the upper phase component (containing a small amount of water) was extracted from time to time via the pipe 409 and recovered in the butanol collection column. The water as the lower phase component (containing a small amount of n-butanol) was refluxed via the pipe 410 to the hydrolyzing can 403. At the time that the n-butanol ceased to exist in the hydrolyzing can 403, the reflux of water from the two phase separator 407 was stopped to allow thorough distillation of the n-butanol. Thereafter, the distillation of the azeotrope from the hydrolyzing can 403 was continued until the amount of water in the azeotrope totalled about 20 ml. Then, the hydrolysis was stopped. The lactic acid obtained in the hydrolyzing can 403 assumed no color and emitted no odor and manifested highly satisfactory thermal stability.

### Example 3

### Pretreatment of culture medium

Corn steep liquor was adjusted to pH 7 with ammonia, boiled for one hour, and subjected to centrifugal separation. Then, fermentation of lactic acid was carried out by following the procedure of Example 1 while using the supernatant produced in consequence of the centrifugal separation as a nutrient.

The fermentation solution was concentrated to a lactic acid concentration of 65% in the same manner as in the concentration of the culture medium for fermentation indicated in (1) of Example 1. The resultant concentrate and n-butanol added thereto in an amount of 2.5 mols per mol of lactic acid were subjected to removal of ammonia in the same manner as in the batchwise removal of ammonia indicated in (2) of Example 1. The final temperature of the interior of the reaction vessel 203 was 131°C. The amount of ammonia contained in the reaction solution which remained after the end of the reaction was 0.064 mol per mol of lactic acid. A black solid substance was found adhering to the part of the inner wall of the reaction vessel 203 near the level of the solution. The adhesion of the black substance to the part of the inner wall below the level of the solution was only slight.

### Example 4

Adaptation of removal of ammonia for continuous operation

A culture medium for fermentation of lactic acid was prepared by using the same culture medium composition and lactic acid bacteria as used in Example 3. It was concentrated to a lactic acid concentration of 63.6% in the same manner as in the concentration of the culture medium for fermentation indicated in (1) of Example 1.

Fig. 5 is a schematic diagram illustrating an apparatus for continuous removal of ammonia used in the present invention.

An apparatus 501 for continuous removal of ammonia, as shown in Fig. 5, was provided with an oil bath 502 and a reaction vessel 503 furnished with stirring vanes and immersed in the oil of the oil bath 502. A distillation column 504 was connected via a pipe 505 to the reaction vessel 503. The distillation column 504 was connected via a forked pipe 508 to a condenser 506 and a two phase separation part 508. The condenser 506 was connected via a pipe 509 to an ammonia absorber (not shown). The two phase separation part 507 was connected via a pipe 510 to the forked pipe 508 so as to reflux the upper phase component of the two phase separation part 507 and then via a pipe 511 to a water recovery column (not shown) so as to recover the lower phase component of the two phase separation part 507. Further, the reaction vessel 503 was connected via a pipe 512 and a pipe 513 respectively to a condensate and an n-butanol storage column (not shown) so as to permit continuous supply of the condensate mentioned above and the n-butanol. The reaction vessel 503 was provided with a pipe 514 extended into the reaction solution so as to allow continuous extraction of the reaction solution and keep the level of the reaction solution at a substantially fixed height. The other end of the pipe 514 was joined to a reaction solution recovery column (not shown) (providing that the provision of this reaction solution recovery column would be omitted and this reaction solution would be forwarded directly to the next step when the method of this invention was operated wholly continuously).

First, 300 ml of the reaction solution obtained after the batchwise removal of ammonia indicated in (2) of Example 1 was placed in the reaction vessel 503. Then, the temperature of the reaction solution was elevated to about 130°C by adjusting the temperature of the oil in the oil bath 502. Thereafter, the concentrate having a lactic acid concentration of 63.6% and n-butanol were respectively forwarded continuously at flow rates of 0.247 mol of whole lactic acid per hour and 0.520 mol of n-butanol per hour via the pipes 512 and 513 to the reaction vessel 503. When the reaction solution in the reaction vessel 503 was heated, the n-butanol formed an azeotrope with water. The azeotrope was collected in the condenser disposed above the top of the distillation column 504 positioned above the reaction vessel 503 and separated into a water phase and a butanol phase in the two phase separation part 507 disposed below the condenser 506. The n-butanol as the upper phase component (containing a small amount of water) was refluxed via the pipe 510 to the forked pipe 508 disposed above the distillation column 504 and the water as the lower phase component (containing a small amount of n-butanol) was extracted from time to time via the pipe 511 and recovered in the water recovery column. An extremely small amount of ammonia gas was collected via the pipe 509 in the ammonia absorber (ice water) without being collected in the condenser 506. The reaction solution was extracted via the pipe 514 from the reaction vessel 503 and recovered in the reaction solution recovery column so as to keep the level of the reaction solution inside the reaction vessel 503 constantly at a fixed height. Samples of the reaction solution extracted via the pipe 514 after 10, 20, and 30 hours following the start of the reaction were found to have ammonia contents of 0.072, 0.088, and 0.076 mol respectively per mol of lactic acid.

During the course of this experiment, a solid substance was found adhering to the part of the inner wall of the reaction vessel 503 near the level of the reaction solution. The deposit of this solid substance on the inner wall did not grow beyond a fixed amount. Absolutely no adhesion of the solid substance was found on the inner wall below the level of the reaction solution.

The results of this working example clearly show that in the substantial absence of water in the reaction solution from beginning, the adhesion of a solid substance to the inner wall of the reaction vessel 503 was noticeably slight as compared with the operation of batchwise removal of ammonia indicated in (2) of Example 1.

### Example 5

Reclamation of water and ammonia for use in fermentation

Ten (10) liters of a culture medium prepared in the same manner as in Example 1 was subjected to fermentation by following the procedure of Example 1. The fermentation lasted for 62 hours. The yield of fermentation was 97.9%. The resultant fermentation broth was concentrated to a lactic acid concentration of 64%. The concentrate and n-butanol added thereto in an amount of 2.5 mols per mol of lactic acid were treated for removal of ammonia in the same manner as in the operation for continuous removal of ammonia indicated in (2) of Example 1. The removed ammonia was collected by being absorbed in distilled water kept cooled with ice in the ammonia absorber. The amount of ammonia remaining in the reaction solution inside the reaction vessel 203 after the end of the esterification was 0.048 mol per mol of lactic acid. The amount of ammonia collected in the ammonia absorber was 6.826 mols per mol of acetic acid. One (1) liter of the culture medium as used in Example 1 were subjected to fermentation of lactic acid by following the procedure of Example 1, except that the recovered water which was obtained after ammonia had been removed by heating from the water recovered during the concentration of the fermentation solution was used as a substitute for the tap water used in Example 1 and the recovered aqua ammonia collecting during the removal of ammonia was used as a neutralizing agent in the place of the 10 mol% aqua ammonia used in Example 1. The fermentation lasted for about 72 hours and the yield of fermentation was 98.0%. These results clearly indicate that the reuse of the recovered aqua ammonia in the fermentation lowered the speed of fermentation slightly but brought about absolutely no effect on the yield.

### Example 6

A reaction for promoting the esterification was carried out under normal pressure in the same manner as in Example 1. After three hours of the esterification, the temperature of the interior of the distillation can 303 reached 130.1°C. At this time, the amount of dibutyl ether produced by the esterification was 0.56% based on the amount of n-butanol charged at first.

When the same operation was carried out under a reduced pressure of 250 mmHg, the reaction lasted for 10 hours and the final temperature of the interior of the distillation can 303 was 101.1°C. At this time, the amount of dibutyl ether produced was only 0.02% based on the amount of n-butanol initially charged.

### Example 7

Fermentation was carried out by faithfully following the procedure of Example 1 except that a culture medium obtained by diluting 100 g of glucose, 20 g of corn steep liquor, and 2 g of yeast extract with tap water to a total volume of 1 liter was used instead. In this working example, the amount of impurities entrained in the fermentation broth based on the amount of lactic acid was smaller than that found in Example 1 because the amount of corn step liquor normally containing impurities copiously was decreased to one half.

The fermentation lasted for 60 hours and the yield of lactic acid was 96.1% based on the amount of glucose. Thereafter, the fermentation broth was concentrated batchwise and subjected to removal of ammonia to promote the esterification in the same manner as in Example 1.

The removal of ammonia was carried out on the fermentation broth charged in an amount of 1.00 mol as reduced to lactic acid. The reaction solution at the end of the reaction was found to contain 0.722 mol of butyl lactate and 0.0653 mol of ammonia.

The promotion of esterification was performed on the fermentation broth charged in an amount of 0.97 mol as reduced to lactic acid. The reaction solution at the end of the reaction was found to contain 0.949 mol of butyl lactate.

In contrast to Example 1 in which the solid component was removed by filtration after the promotion of the esterification, the present example directly distilled the solution containing the solid component because the amount of impurities contained therein was small owing to the aforementioned change in the composition of the culture medium for fermentation.

The distillation of butyl lactate was carried out by following the procedure of Example 1. The amount of butyl lactate in the reaction solution subjected to the promotion of the esterification was 0.92 mol as reduced to lactic acid. After the end of the distillation, the amount of butyl lactate was 0.888 mol. Thus, the yield of distillation was 97.7%.

The residue in the distillation can 303 showed ample flowability at a temperature in the range of 50° to 60°C. Absolutely no sign of adhesion of a solid substance to the inner wall of the distillation was 303 was detected.

### Control 1

Case of omitting removal of ammonia

The same fermentation broth as used in Example 7 was subjected to the following experiment.

In the reaction vessel 203 illustrated in Fig. 2, 204.20 g of a concentrate of a fermentation broth (1.224 mols as reduced to lactic acid) and 272.3 g of n-butanol (3.672 mols) were stirred and meanwhile adjusted to pH 1.0 by gradual addition of 66.83 g of concentrated sulfuric acid (0.667 mol). The resultant mixture was thermally dehydrated to undergo accelerated esterification. The esterification lasted for three hours. At the end of the fermentation, the temperature of the reaction solution reached 132°C and the reaction solution was found to have formed 1.137 mols of butyl lactate.

The esterification solution was passed through a filter paper. The solid component (mainly of ammonium sulfate) was washed with n-butanol and then dried. The dried solid component weighed 83.96 g (0.802 part per part of lactic acid).

The esterification solution remaining after the removal of the solid component mentioned above (1.03 mols as reduced to lactic acid) was distilled by use of the apparatus for distillation illustrated in Fig. 3 in the same manner as the distillation of butyl lactate indicated in Example 1. As a result, 0.912 mol of butyl lactate was obtained. The yield of distillation of butyl lactate was 98.2%.

The residue in the distillation can 303 retained flowability while the distillation was proceeding. When the supply of raw materials was stopped, the residue showed viscosity so high as to defy agitation and was found adhering to the inner wall of the distillation can 303.

## Claims

1. A method for the production of a lactic ester involving the fermentation of lactic acid with a microorganism, the method comprising: (a) carrying out the adjustment of the pH of a culture medium undergoing the fermentation of lactic acid with ammonia; (b) adding an alcohol having four or five carbon atoms to at least one solution selected from a solution of ammonium lactate obtained by the fermentation, a concentrate of the solution of ammonium lactate mentioned above, and the solution resulting from the separation of solid substances including the microorganic cells used for the fermentation from either of the said solutions, and heating the resultant mixture thereby including dehydration and esterification of lactic acid with the alcohol while, at the same time, effecting liberation and recovery of ammonia; and (c) adding a mineral acid to the solution obtained by the esterification reaction of (b) and heating and dehydrating the resultant mixture in an acidic state thereby promoting and completing the esterification of lactic acid with said alcohol.

2. A method as claimed in Claim 1, characterised in that the amount of the alcohol added is in the range of 1 to 10 mols per mol of lactic acid.

3. A method as claimed in Claim 1 or Claim 2, characterised in that the alcohol is n-butanol.

4. A method as claimed in any preceding Claim, characterised in that the solution is the concentrate of an ammonium lactate solution obtained by the fermentation.

5. A method as claimed in Claim 4, characterised in that the concentrate has a lactic acid concentration in the range of 60 to 75% by weight.

6. A method as claimed in any preceding Claim, characterised in that the reaction temperature in the esterification step (b) is in the range of 100° to 170°C.

7. A method as claimed in any preceding Claim, characterised in that the reaction in step (c) for promoting esterification is carried out under a pressure in the range of 100 mmHg and 760 mmHg (1.33 kPa and 98.1 kPa).

8. A method as claimed in any preceding Claim, characterised in that the reaction in step (c) for promoting the esterification is carried out at a temperature in the range of 100° to 160°C.

9. A method for the production of lactic acid, characterised in that it comprises producing a lactic ester by a method as claimed in any preceding Claim and subsequently hydrolysing the resultant lactic ester.

10. A method as claimed in Claim 9, characterised in that the hydrolysis is carried out by using an acid catalyst.

11. A method as claimed in Claim 10, characterised in that the acid catalyst is an ion-exchange resin or a mineral acid.
